# NEW EUROPEAN PATENT SPECIFICATION

(11) **EP 0 512 635 B2**
(45) Date of publication and mention of the opposition decision: **29.11.2000**
(45) Mention of the grant of the patent: 22.01.1997
(21) Application number: 92201247.1
(22) Date of filing: 04.05.1992
(51) Int. Cl.: C07C 1/08, C07C 1/04

(54) **A process for the production of isoparaffins**
Verfahren zur Herstellung von Isoparaffinen
Procédé pour la fabrication des isoparaffines

(30) Priority: 07.05.1991 GB 9109747
(43) Date of publication of application: 11.11.1992
(73) Proprietor: SHELL INTERNATIONALE RESEARCH MAATSCHAPPIJ B.V., 2596 HR Den Haag (NL)
(72) Inventor: Sie, Swan Tiong, NL-1031 CM Amsterdam (NL)

(56) References cited:
- EP-A- 0 127 220
- EP-A- 0 147 873
- EP-A- 0 256 604
- UA-A- 4 210 771
- US-A- 3 755 144
- US-A- 4 176 053
- US-A- 4 385 193
- US-A- 4 423 265
- US-A- 4 522 939
- US-A- 4 709 116
- US-A- 4 804 802
- US-A- 4 855 529
- US-A- 4 956 521
- US-A- 5 292 983
- J. EILERS et al, The Shell Middle Distillate Synthesis Process (SMDS), Catalyst Letters, 7, March 1990, 253-270
- M. F. SYMONIAK et al, Isomerize Light Naphthas, Paper no. AM-83.57, National Petroleum Refiners' Association Meeting, San Francisco, March 20-23, 1983
- M. F. SYMONIAK, Processing Light Naphtha: The Last Virgin, May1980
- Encyclopedia of Chemical Technology, Kirk Othmer, 1978, John Wiley & Sons, pp. 544-581
- Erdöl, Ergas, Kohle, July/August 1986, The Shell Middle Distillate Synthesis Process, M. J. v.d.Burgt et al, pp. 351-354
- ACS Symposium, September 20, 1975, 1-27, J. Weitkamp, The Influence of Chain Length in Hydrocracking and Hydroisomerization of n-Alkanes

## Description

The invention relates to a process for the production of isoparaffins having 4 to 7 carbon atoms per molecule from synthesis gas.

It is known in the art to prepare heavy paraffins-containing hydrocarbon mixtures by means of the Fischer-Tropsch synthesis. Recently, it has been found advantageous to subject the hydrocarbons so-produced to an isomerization/hydrocracking treatment to yield hydrocarbon components valuable as fuels. Thus, United Kingdom patent No. 2,077,289 discloses a process comprising contacting a mixture of carbon monoxide and hydrogen, also known as synthesis gas, with a catalyst active in the Fischer Tropsch synthesis and thereafter cracking the resulting paraffinic hydrocarbons in the presence of hydrogen to yield middle distillate fuels. A similar process scheme is disclosed in European patent No. 0 147 873.

A most interesting class of hydrocarbons, finding particular use as components in gasoline blending, are isoparaffins having from 4 to 7 carbon atoms. Surprisingly, a process has been found allowing isoparaffins in this class to be prepared from synthesis gas in high yields.

Accordingly, the present invention provides a process for the preparation of isoparaffins having from 4 to 7 carbon atoms per molecule from synthesis gas, which process has been described in the claims.

Fischer-Tropsch hydrocarbon synthesis catalysts usually contain one or more metals of the iron group, preferably cobalt, together with one or more promoters and a carrier material.

In the process of the invention it is preferred to use in the first step the cobalt catalysts which form the subject matter of European patent application publication No. 0 127 220. These catalysts contain 3-60 pbw cobalt and 0.1-100 pbw of at least one other metal chosen from the group formed by zirconium, titanium, rhenium, ruthenium and chromium per 100 pbw silica, alumina or silica-alumina and/or titania.

These catalysts have advantageously been prepared by kneading and/or impregnation. They preferably satisfy the relation:$\text{(3+4R) >} \frac{\text{L}}{\text{S}} \text{> (0.3+0.4R),}$ wherein
L is the total quantity of cobalt present on the catalyst, expressed as mg Co/ml catalyst,
S is the surface area of the catalyst, expressed as m²/ml catalyst, and
R is the weight ratio of the quantity of cobalt deposited on the catalyst by kneading to the total quantity of cobalt present on the catalyst.

The preparation of the cobalt catalysts which are used in the first step of the process of the invention is preferably carried out according to one of the three procedures mentioned hereinafter:
a) first cobalt is deposited in one or more steps by impregnation and subsequently the other metal is deposited in one or more steps, also by impregnation,
b) first the other metal is deposited in one or more steps by impregnation and subsequently the cobalt is deposited in one or more steps, also by impregnation, and
c) first cobalt is deposited in one or more steps by kneading and subsequently the other metal is deposited in one or more steps by impregnation.

In the process according to the invention preference is given to the use of cobalt catalysts containing 15-50 pbw cobalt per 100 pbw carrier. The preferred quantity of other metal present in the cobalt catalysts depends on the way in which this metal has been deposited. In the case of catalysts where first cobalt has been deposited on the carrier, followed by the other metal, preference is given to catalysts containing 0.1-5 pbw other metal per 100 pbw carrier. In the case of catalysts where first the other metal has been deposited on the carrier, followed by the cobalt, preference is given to catalysts containing 5-40 pbw of the other metal per 100 pbw carrier. Preference is given to zirconium as the other metal and to silica as carrier material. In order to be suitable for use the cobalt catalysts should first be reduced. This reduction may suitably be carried out by contacting the catalyst at a temperature between 200 and 350 °C with a hydrogen-containing gas.

In the process of the invention the hydrocarbon synthesis of the first step is preferably carried out at a temperature of 125-350 °C and in particular of 175-275 °C. The first step of the process is preferably carried out at a pressure of 5-100 bar and in particular of 10-75 bar.

H₂/CO mixtures which are eligible to be used for the preparation of isoparaffins according to the invention can very suitably be obtained, starting from a heavy carbonaceous material such as coal, or starting from light hydrocarbons such as natural gas, by steam reforming or partial oxidation or a combination of these processes. Since the H₂/CO consumption ratio of the preferred cobalt catalysts is about 2, in the process according to the invention, in those cases where the highest possible CO conversion is aimed at, it is preferred to use H₂/CO mixtures having a H₂/CO molar ratio higher than 2. Very suitable for carrying out the first step of the present process is a feed which can be obtained in the steam reforming of natural gas, which yields H₂/CO mixtures having a H₂/CO molar ratio of about 3, as is well-known. If the feed for the first step of the process of the invention available is a H₂/CO mixture having a H₂/CO molar ratio of 2 or less, then the H₂/CO molar ratio of said feed can be increased preparatory to contacting the feed with the cobalt catalysts. Such an increase of the H₂/CO molar ratio may be brought about by, inter alia, addition of hydrogen, removal of carbon monoxide, mixing with a hydrogen-rich H₂/CO mixture or by subjecting the low-hydrogen feed to the CO-shift reaction. Optionally the CO-shift reaction can be carried out in situ by addition in the first step of the process of a physical mixture of a CO-shift catalyst and the cobalt catalyst. It is also possible to subject low-hydrogen synthesis gases to the process according to the invention without raising their H₂/CO molar ratios. Unconverted hydrogen and carbon monoxide remaining in the effluent of the first step may be recirculated to once again contact the catalyst. In this way, the gas contacting the catalyst may have a H₂/CO ratio substantially lower than that of the feed gas.

The first step of the present process is followed by a second step in which at least part of the naphtha fraction produced in the first step is hydrocracked and isomerized. In the second step a catalyst is used which preferably contains a catalytically active metal component. This metal component can be deposited on any acid carrier having cracking and isomerization activity, for example a chlorided alumina or zeolitic carrier. A zeolitic carrier is preferred.

The catalyst used in the second step of the process according to the invention may contain as catalytically active metal components one or more metals selected from Groups VIB, VIIB and/or VIII of the Periodic System. Examples of such metals are molybdenum, tungsten, rhenium, the metals of the iron group and the metals of the platinum and palladium groups. Catalysts with a noble metal as catalytically active metal component generally contain 0.05-5 parts by weight and preferably 0.1-2 parts by weight of metal per 100 parts by weight of carrier material. Very suitable noble metals are palladium and platinum. Catalysts with a non-noble metal or a combination of non-noble metals as catalytically active metal component generally contain 0.1-35 parts by weight of metal or combination of metals per 100 parts by weight of carrier material. Very suitable hydrocracking catalysts contain a combination of 0.5-20 parts by weight and in particular 1-10 parts by weight of a non-noble metal of Group VIII and 1-30 parts by weight and in particular 2-20 parts by weight of a metal of Group VIB and/or VIIB per 100 parts by weight of carrier material. Particularly suitable metal combinations are combinations of nickel and/or cobalt with tungsten and/or molybdenum and/or rhenium. Likewise very suitable as hydrocracking catalysts are catalysts which contain 0.1-35 parts by weight and in particular 1-15 parts by weight of nickel per 100 parts by weight of carrier material.

If the present hydrocracking catalysts contain a non-noble metal or combination of non-noble metals as catalytically active metal component, they are preferably used in their sulphidic form. The conversion of the hydrocracking catalysts to their sulphidic form can very suitably be carried out by contacting the catalysts at a temperature below 500 °C with a mixture of hydrogen and hydrogen sulphide in a volume ratio of 5:1 to 15:1. The conversion of the catalysts into the sulphidic form may also be carried out by adding to the feed, under reaction conditions, sulphur compounds in a quantity of from 10 ppmw to 5% by weight and in particular in a quantity of from 100 ppmw to 2.5% by weight.

The isomerization/hydrocracking step (b) of the present process is preferably carried out using a catalyst comprising a zeolite having a pore diameter in the range from 0.5 to 1.0 µm. The silica:alumina ratio of the zeolite is preferably in the range from 5 to 200.

Very suitable zeolites for this purpose are zeolite Y, mordenite, zeolite β and ZSM-20.

Both the natural and the synthetic zeolites often contain a certain quantity of alkali-metal ions. In order to make the zeolites more suitable for use as carrier material for catalysts in hydrocarbon-conversion processes, it is preferred to replace at least part of the alkali-metal ions present therein by other cations, in particular by hydrogen ions and/or ammonium ions and/or ions of the rare-earth metals. For use as carrier for catalysts in the hydrocarbon conversion process of step (b), the preference is for zeolites with an alkali-metal content of less than 1% by weight, in particular of less than 0.1% by weight.

The metals can be applied to the zeolite in any conventional manner such as by impregnation, percolation or ion exchange. After the catalytically active metal components have been applied to the carrier, the catalyst is usually dried and subsequently calcined. Hydroconversion catalysts are usually employed in the form of particles with a diameter of 0.5-5 mm. However, zeolites suitable for use as carrier material for the present hydroconversion catalysts are often available as a fine powder. The zeolites may be shaped into particles of larger dimensions, for example, by compression and extrusion. Shaping is preferably carried out after the catalytically active metal components have been applied, but before the calcination during which the metals are converted into the corresponding oxides. During shaping the zeolite may, if desired, be combined with an inorganic matrix or binder. Examples of suitable matrices or binders are natural clays and synthetic inorganic oxides. Prior to the formation of the shaped particles the zeolite and the matrix or binder should first be homogeneously mixed. The shaped particles may have any desired form, such as pearls, spheres, pellets, tablets, briquettes and granules.

If, in the preparation of the present hydroconversion catalysts, use is made of a matrix or binder, it is preferred to prepare shaped particles which contain 10-90% by weight of the matrix or binder and by preference use is made of a matrix or binder that does not contain any alkali metals or whose alkali metal content is very low. If desired, mixtures of zeolites may be used as carrier material for the present hydroconversion catalysts. Such zeolitic mixtures may also be used in combination with a matrix or binder.

Suitable conditions for the hydrocracking/isomerization of the heavy paraffins-containing hydrocarbon mixture according to the second step of the process according to the invention are a temperature of 250 °C-450 °C, a pressure of 5-50 bar, an hourly space velocity of 0.2-20 kg of hydrocarbon feed per kg of catalyst per hour and a hydrogen/hydrocarbon feed molar ratio of 1-50. It is preferred to carry out the isomerization/hydrocracking step (b) at a temperature of 250 °C-350 °C and a pressure of 7-15 bar.

Advantageously at least part of the effluent of the isomerization/hydrocracking step (b) is passed to an additional separation step in which a hydrogen-containing gas and a hydrocarbon effluent are separated from each other. Suitably, in this separation step a hydrogen-containing gas and a hydrocarbon effluent are separated off by flash distillation. Suitably the flash distillation is carried out at a temperature between -20 and 100 °C, and a pressure between 1 and 50 bar.

Preferably at least part of the liquid hydrocarbon product of step (b) of the process according to the invention is separated by fractional distillation to obtain a C₇- fraction and a C₈+ fraction. At least part of the C₈+ fraction is advantageously recycled to step (b). At least part of the C₇- fraction is passed to step (c).

In separation step (c), preferably a separatory molecular sieve is used capable of adsorbing normal hydrocarbons. Suitably, unbranched hydrocarbons are substantially adsorbed, whereas branched hydrocarbons are not retained in any substantial amount by the molecular sieve and are separated off as a product stream. This selectivity is dependent to a large extent on the pore diameters of the molecular sieve, which diameters are preferably in the range from 0.3-0.8 nm, and most preferably from 0.4-0.6 nm. Suitably the hydrocarbon separation step comprises a separatory molecular sieve having a pore size which is sufficient to permit entry of normal hydrocarbons containing 4-7 carbon atoms, but restrictive to prohibit entry of such mono-methyl branched, and dimethyl branched hydrocarbons.

Suitably, synthetic or natural zeolites, erionite and offretite are used as molecular sieve, and preferably zeolite 5A. The particles which comprise molecular sieve material may in addition comprise a binder material such as alumina, silica or silica-alumina, in order to improve the crushing strength of the particles; said particles may also be mixed with particles which do not contain molecular sieve material.

The adsorption in step (c) is advantageously carried out at a temperature in the range from 100 to 300 °C and at a pressure in the range from 5 to 25 bar.

The hydrocarbons which are adsorbed on the separatory molecular sieve in step (c), can be desorbed therefrom by contacting the sieve with a liquid solvent or with a gas. Preferably, in step (c) adsorbed normal hydrocarbons are desorbed by passing a hydrogen-containing gas over the molecular sieve and advantageously at least part of the hydrogen- and normal hydrocarbons-containing effluent of step (c) is passed to step (b).

Hydrocarbons which are adsorbed on the separatory molecular sieve, can be periodically desorbed therefrom by interrupting the stream of hydrocarbon effluent and passing a hydrogen-containing gas over the separatory molecular sieve. After desorption of a substantial amount of the adsorbed hydrocarbons, the stream of hydrogen-containing gas is interrupted and the stream of hydrocarbon effluent from the isomerization step is contacted again with the sieve. It is possible to carry out the process such that a continuous stream containing normal hydrocarbons is obtained, e.g. by using several vessels. The hydrogen-containing gas which can be used for desorbing the adsorbed hydrocarbons, need not be completely pure and may contain a certain amount of other components, suitably up to 40 mol % and preferably not more than 20 mol % of other compounds such as hydrocarbons, e.g. reformer off-gas, provided that these compounds are substantially inert with respect to the feed and the separatory molecular sieve applied. The hydrogen-containing gas is suitably passed over the molecular sieve at a temperature of between 200 and 450 °C and a pressure of between 1 and 25 bar.

The process according to the present invention can be carried out in a number of alternative ways, and a preferred process scheme according to the present invention will be elucidated more fully hereinafter, with reference to the accompanying figures, to which the invention is by no means restricted. The processes depicted in the figures are carried out with the help of an isomerization/hydrocracking reactor (10), a product separator (20), a fractionator (30), a recycle gas compressor (40) and three iso/normal paraffins separators (501), (502) and (503).

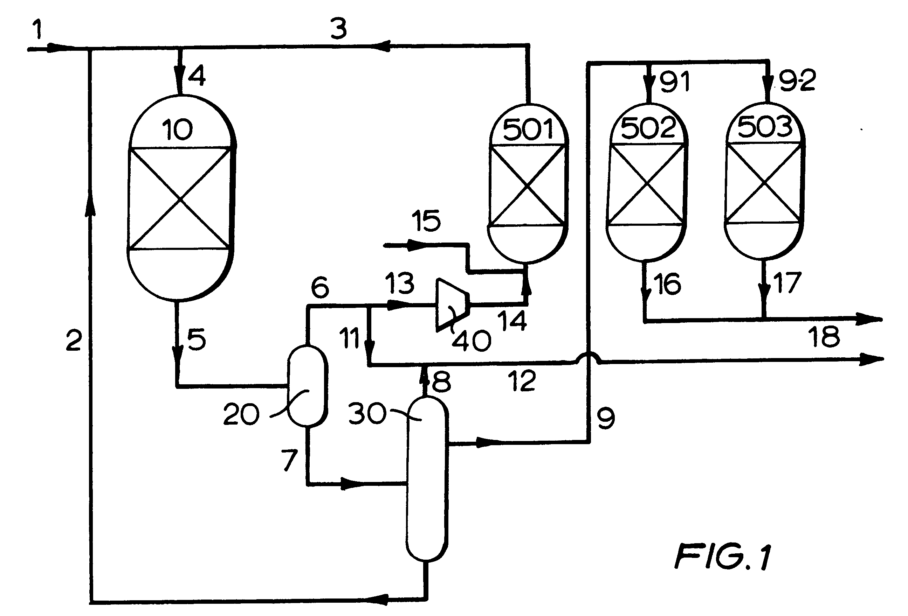

In the process schematically shown in figure 1 (not according to the invention) gasoline-range isoparaffins are produced by converting the effluent from a Fischer-Tropsch hydrocarbon synthesis step (not shown). To this end the Fischer-Tropsch effluent is introduced into the process via a line (1). It is combined with a C₈+ recycle stream obtained via a line (2) from the fractionator (30), and with a normal paraffins and hydrogen-containing recycle stream obtained via a line (3) from the separator (501). The combined streams (1), (2) and (3) are introduced via a line (4) into the isomerization/hydrocracking reactor (10) wherein higher boiling normal paraffins are at least in part hydrocracked and lower boiling normal paraffins are at least in part isomerized in the presence of hydrogen.

The effluent from the reactor (10) is passed via a line (5) to the product separator (20) where it is separated into a hydrogen-rich gaseous stream which is withdrawn via a line (6), and a hydrocarbons-containing liquid stream which is passed through a line (7) to the fractionator (30) where it is separated into a light hydrocarbons (predominantly C₃-) stream which is withdrawn from the fractionator (30) via a line (8), a C₄-C₇ iso/normal paraffins-containing stream which is transferred via a line (9), a line (91) and a line (92) to the iso/normal paraffins separators (502) and (503), and a C₈+ hydrocarbons-containing recycle stream which is recycled to the reactor (10) via the lines (2) and (4). The hydrogen-containing stream which passes through the line (6) is divided. One part is passed through a line (11) to be combined with the light hydrocarbons-containing gaseous stream in the line (8) and withdrawn from the system via a line (12).

The remaining part of the stream from the line (6) is passed via a line (13), to the recycle gas compressor (40) and a line (14) to the iso/normal paraffins separator (501). Via a line (15) make-up hydrogen is supplied to the line (14).

In the iso/normal paraffins separators (501), (502) and (503) the iso- and normal C₄-C₇ paraffins-containing mixture is separated into a C₅-C₇ isoparaffins- plus i- and n-C₄ paraffins-containing stream and a C₅-C₇ normal paraffins-containing stream. To this end the mixture is passed over two separators, normal C₅-C₇ paraffins being adsorbed on special zeolite beds and isoparaffins plus normal C₄ paraffins passing through these beds.

At the same time normal paraffins are desorbed from one or more zeolite beds contained in a third separator by passing a hydrogen-containing gas over the zeolite beds. Intermittently the separators are used for adsorption and desorption. In this way normal C₅-C₇ paraffins are desorbed from the content of separator (501) by passing the hydrogen-containing gas supplied via the line (14) thereover. The normal C₅-C₇ paraffins and hydrogen-containing mixture is withdrawn from the separator (501) and recycled via the lines (3) and (4) to the isomerization/hydrocracking reactor (10).

C₄-C₇ isoparaffins and normal C₄ paraffins not being adsorbed by the zeolite beds in separators (502) and (503) pass through these separators and are withdrawn as a product from the system via a line (16), a line (17) and a line (18) respectively.

This C₄-C₇ isoparaffins and C₄ normal paraffins mixture is preferably separated into normal butane and iso-butane and a C₅-C₇ isoparaffins mixture. The latter is an excellent motor gasoline blending component having a high octane number and a desired volatility. The iso-butane can be used for the production of C₇ and C₈ isoparaffins by alkylation, and of methyl tertiary butyl ether and tertiary butyl alcohol. These products are also excellent motor gasoline blending components. N-butane can also be blended into the gasoline up to the limits set by vapour pressure specifications.

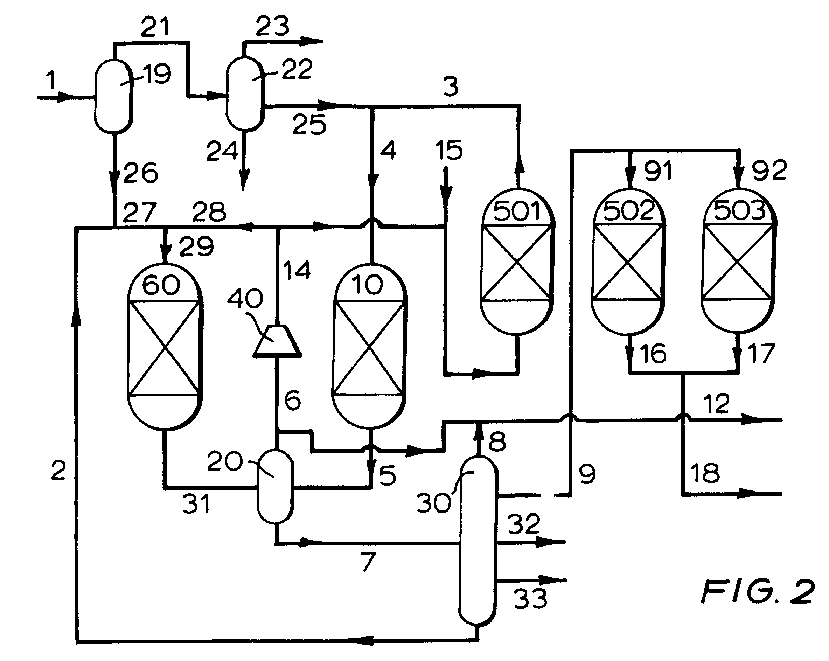

In figure 2 a process according to the invention is schematically shown for the production of a C₄-C₇ isoparaffins-containing gasoline blending component, a C₃ and C₄ olefins-containing chemical feedstock, a kerosine fraction and a gas oil fraction.

To this end the effluent from a Fischer-Tropsch hydrocarbon synthesis step i.e. a heavy paraffins-containing hydrocarbon mixture is introduced into the process via a line (1) and passed to a high temperature separator (19) operating at a temperature in the range from 200 to 350 °C and a pressure in the range from 20 to 40 bar. In this separator (19) the Fischer-Tropsch effluent is separated into a gaseous stream containing hydrogen and light (e.g. C₁₀-) hydrocarbons and a heavy liquid fraction boiling above 150 °C and containing C₁₀+ hydrocarbons. The gaseous stream is cooled and partially condensed in a cooler (not shown) and passed through a line (21) to a low temperature separator (22) wherein it is separated into gas containing hydrogen and mainly C₁-C₄ hydrocarbons, a liquid aqueous stream which is withdrawn from the system via a line (24) and a liquid hydrocarbon stream comprising mainly C₄+ paraffins. The gas is highly olefinic and therefore a desired feedstock for the chemical industry or for the production of highly branched iso-paraffins by alkylation. It is withdrawn from the system via a line (23).

The liquid hydrocarbon stream comprising paraffins is passed through a line (25) and a line (4) to an isomerization/hydrocracking reactor (10).

The heavy liquid stream obtained from the separator (19) and comprising C₁₀+ hydrocarbons is combined via a line (26) with a bottoms recycle stream passed via a line (2) from the fractionator (30) and containing C₂₀+ paraffins.

The combined streams are further combined via a line (27) with a hydrogen-containing gaseous stream transferred via a line (6), a recycle gas compressor (40), a line (14) and a line (28). The combined liquid and gaseous streams are passed via a line (29) to a heavy paraffins hydrocracking reactor (60). In this reactor heavy paraffins are hydrocracked at a temperature in the range from 250 to 400 °C and pressure in the range from 20 to 50 bar. Preferably, a catalyst is used which contains 0.1-2% by weight, and in particular 0.2-1% by weight, of one or more noble metals of Group VIII supported on a carrier. Preference is given to catalysts containing platinum or palladium as noble metal. Examples of suitable carriers for the noble metal catalysts are amorphous oxides of the elements of Groups II, III and IV, such as silica, alumina, magnesia and zirconia and also mixtures of these oxides, such as silica-alumina, silica-magnesia and silica-zirconia. Preferred carriers for the noble metal catalysts are silica-aluminas.

The effluent from the hydrocracking reactor (60) is passed through a line (31) to a product separator (20) where it is combined with the effluent from the isomerization/hydrocracking reactor (10) in which the liquid hydrocarbon stream from the line (4) has been isomerized and hydrocracked using hydrogen-containing gas which has been obtained via a line (3) from an iso/normal paraffin separator (501).

In the separator (20) the combined streams are separated into a hydrogen-rich gaseous stream and a higher boiling hydrocarbons-containing liquid stream.

The gaseous stream is recycled in part through the lines (6), (14), (28) and (29), by the recycle gas compressor (40) to the hydrocracking reactor (60) while a bleed stream from this gaseous stream is withdrawn from the system via a line (12). The liquid stream is passed through a line (7) to the fractionator (30) where it is separated into a gas containing traces of hydrogen and C₁-C₃ hydrocarbons, a liquid C₄-C₇ containing light naphtha stream, a kerosine stream (boiling in the range from 150 to 250 °C) and a gas oil stream (boiling in the range from 250 to 370 °C).

The gas is withdrawn from the system via a line (8) and the line (12). The kerosine and gas oil are withdrawn as products from the system via a line (32) and a line (33) respectively. Because these products consist mainly of paraffins they are of excellent quality.

The light naphtha stream is passed via a line (9), a line (91) and a line (92) to the iso/normal paraffins separators (502) and (503) where an isoparaffins (C₄-C₇) and normal butane-containing stream is separated therefrom which is an excellent gasoline blending component. It is withdrawn from the system via the lines (16), (17) and (18), while normal C₅-C₇ paraffins are being adsorbed on the zeolite beds contained in the separators (502) and (503).

In a following desorption cycle the normal paraffins are desorbed from the zeolite beds as is the case with the normal paraffins having been adsorbed in separator (501) in a former adsorption cycle (not shown). In the scheme shown in Fig. 2 hydrogen-containing gas is passed through the lines (14) and (15) and adsorber (501) thereby desorbing normal paraffins from the zeolite bed(s) contained in the adsorber (501) which normal paraffins are recycled through the lines (3) and (4) to isomerization/hydrocracking reactor (10).

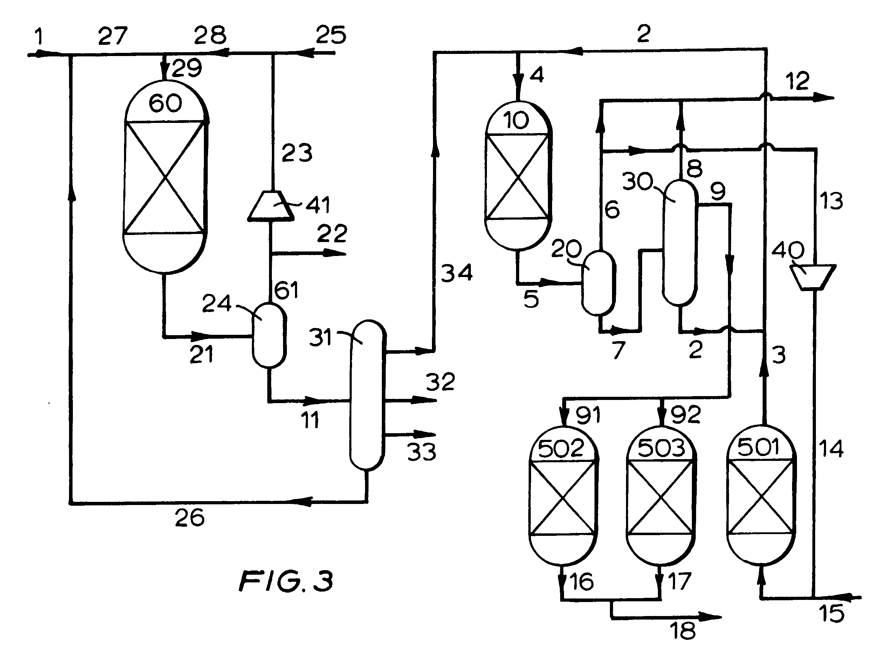

In figure 3 a process is schematically shown for the production of high octane C₄-C₇ isoparaffin gasoline components, high cetane gas oil and low smoke point kerosine from a heavy paraffinic synthetic hydrocarbon product. The latter is introduced into the process via a line (1) and combined with a heavy paraffins (C₂₀+)-containing recycle stream transferred from a fractionator (31) through a line (26). The combined streams are passed through a line (27) and combined with a hydrogen and light (mainly C₁-C₃) hydrocarbon-containing gas supplied via a line (28). The combined streams are passed via a line (29) to a heavy paraffins hydrocracking reactor (60).

In this reactor the heavy paraffins are hydrocracked using a similar catalyst and reaction conditions as described hereinbefore with reference to the reactor (60) of figure 2.

The effluent from the reactor (60) is passed via a line (21) to a product separator (24) where it is separated into a hydrogen and light hydrocarbons (mainly C₁-C₃)-containing gas and a higher boiling hydrocarbons-containing liquid stream containing hydrocarbons from C₄ upward. The gas is in part recycled through a line (61), a recycle gas compressor (41) and the lines (28) and (29) to the reactor (60), make-up hydrogen being supplied via a line (25). The remaining part of the gas is withdrawn from the system as a bleed stream via a line (22).

The liquid stream is passed via a line (11) to the fractionator (31) by means of which it is separated into a paraffinic naphtha (C₄ to C₁₀), a kerosine (boiling in the range from 150 to 250 °C and having a smoke point of at least 40 mm), a gas oil (boiling in the range from 250 to 370 °C and having a cetane number of at least 60) and a heavy paraffins-containing bottom product (boiling above 370 °C). The kerosine and the gas oil are withdrawn from the process as final products via a line (32) and a line (33) respectively. The bottom product is recycled to the reactor (60) via the lines (26), (27) and (29).

The paraffinic naphtha is passed via a line (34) to a line (4) wherein it is combined with a hydrogen and normal paraffins-containing recycle stream supplied via a line (2). The combined streams (34) and (2) are introduced via the line (4) into the isomerization/hydrocracking reactor (10) wherein higher boiling normal paraffins are at least in part catalytically hydrocracked and lower boiling normal paraffins are at least in part catalytically isomerized in the presence of hydrogen.

The effluent from reactor (10) is passed via a line (5) to product separator (20) where it is separated into a hydrogen and light hydrocarbons (mainly C₁-C₃)-containing gaseous stream and a higher boiling hydrocarbons (C₄-C₁₀)-containing liquid stream. The gaseous stream is withdrawn via a line (6). It is partly removed from the system via a line (12) and partly recycled to an iso/normal paraffins separator via a line (13), the recycle gas compressor (40) and a line (14).

The liquid stream is passed through a line (7) to the fractionator (30) wherein it is separated into a light hydrocarbons (mainly C₃)-containing gas stream, a C₄-C₇ iso/normal paraffins-containing liquid stream and a C₈+ hydrocarbons-containing liquid stream. The gas stream is withdrawn from the system via a line (8) and a line (12).

The C₈+ hydrocarbons-containing liquid stream is recycled via a line (2), wherein it is combined with a normal C₅-C₇ paraffins-containing stream from a line (3), and the line (4) to the isomerization/hydrocracking reactor (10).

The C₄-C₇ iso/normal paraffins-containing liquid stream is passed via lines (9), (91) and (92) to iso/normal paraffins separators (502) and (503).

In these separators normal C₅-C₇ paraffins are adsorbed on zeolite beds, while C₄-C₇ isoparaffins and C₄ normal paraffins pass through these beds and are withdrawn from the system as final products via lines (16), (17) and (18). At the same time normal C₅-C₇ paraffins are desorbed from one or more zeolite beds contained in an iso/normal paraffins separator (501) to which they had been adsorbed during a former process cycle (not shown). The desorption is effected by passing the light hydrocarbons and hydrogen-containing stream in line (14) over the zeolite bed(s). Fresh make-up hydrogen is introduced to the system via a line (15).

A gaseous mixture of hydrogen, light hydrocarbons and desorbed C₅-C₇ normal paraffins is recycled via a line (3) and the lines (2) and (4) to the isomerization/hydrocracking reactor (10).

The following reference example is given:

In a process as shown in figure 1, 100 t/d of a hydrocarbon product from a Fischer-Tropsch synthesis plant (not shown) are introduced via the line (1) together with 80 t/d of a recycle stream (2), mainly consisting of C₈+ paraffins and a gas comprising 15 t/d hydrogen and 25 t/d recycled normal C₄-C₇ paraffin supplied via the line (3), via the line (4) to the isomerization/hydrocracking reactor (10).

The Fischer-Tropsch product is obtained from synthesis gas having a H₂/CO ratio of 2/1 using a catalyst consisting of 30 pbw Co, 12 pbw Zr deposited on 100 pbw SiO₂ by impregnation. The conditions during the hydrocarbon synthesis are:

| | |
|---|---|
| Temperature | 220 °C |
| Pressure | 30 bar |
| Space velocity | 600 nm³/nm³/hr. |

The Fischer-Tropsch product consists mainly of paraffins boiling in the range from 20 to 500 °C and for 80% by weight of heavy paraffins boiling in the range from 150 to 500 °C.

In the isomerization/hydrocracking reactor (10) the Fischer-Tropsch effluent is converted to a lower boiling iso/normal paraffins-containing mixture. In the reactor (10) a catalyst is used consisting of 0.4 pbw Pt deposited on 100 pbw zeolite Y being in the hydrogen form and having a Na content of 0.05% by weight. The reaction conditions are:

| | |
|---|---|
| Temperature | 300 °C |
| Pressure | 20 bar |
| Partial hydrogen pressure | 15 bar |
| Space velocity | 1 kg/kg/hr. |

The isomerization/hydrocracking product is separated in separator (20) in a hydrogen-rich gas and 203 t/d condensate and the latter is further separated in fractionator (30) into 13 t/d of a light hydrocarbons-containing bleed stream (12), 110 t/d of a C₄-C₇ iso/normal paraffins-containing stream (9) and 80 t/d of a heavy paraffins-containing recycle stream (2).

To the hydrogen and light hydrocarbons-containing gas 2.5 t/d fresh hydrogen make-up gas is added via the line (15) and the resulting gas mixture is passed over a zeolite bed contained in adsorber (501) to desorb 25 t/d normal C₅-C₇ paraffins which are recycled via the lines (3) and (4) to the reactor (10).

From the C₄-C₇ iso/normal paraffins-containing stream (9) 85 t/d of a C₄-C₇ isoparaffins- and C₄ normal paraffins-containing mixture is separated by passing stream (9) over 2 zeolite beds, comprising zeolite (5A) as an absorbent and contained in absorbers (502) and (503). The isoparaffins and nC₄ paraffins mixture is removed from the system via the lines (16), (17) and (18) as a final product.

This mixture consists of:

| | |
|---|---|
| nC₄ + iC₄ | 23% by weight |
| iC₅ | 40% by weight |
| iC₆ | 29% by weight |
| iC₇ | 8% by weight. |

It is separated into an i-butane product, a n-butane product and a C₅-C₇ isoparaffins mixture. The iC₄ paraffins are useful as a feedstock for an alkylation process.

The C₅-C₇ isoparaffins are useful as a motor gasoline blending component having a RON* of 86 and a MON** of 85.
* RON = Octane number determined by the research method.
** MON = Octane number determined by the motor method.

## Claims

1. A process for the production of isoparaffins having 4 to 7 carbon atoms per molecule from synthesis gas comprising the following steps:
a) Synthesis of a heavy paraffins-containing hydrocarbon mixture over a Fischer-Tropsch catalyst; hydrocracking at least part of the heavy paraffins-containing hydrocarbon mixture in a hydrocracking reactor; separating the effluent of the hydrocracking reactor into a hydrogen-containing gas which is at least in part recycled to the hydrocracking reactor, a naphtha fraction which is at least in part passed to step (b), a kerosene fraction and a gas oil fraction which are withdrawn as products from the process, and a heavy fraction which is at least in part recycled to the hydrocracking step;
b) conversion of at least part of the naphtha fraction of step (a) over a bi-functional isomerization/hydrocracking catalyst under isomerization/hydrocracking conditions;
c) separation of normal paraffins from at least part of the isoparaffins-containing product of step (b);
d) recycling at least part of the normal paraffins to step (b) and withdrawing C4-C7 isoparaffins as a product from the process.

2. A process as claimed in claim 1, in which in step (a) a Fischer-Tropsch catalyst is used, comprising cobalt.

3. A process as claimed in claim 2, wherein the catalyst comprises 3-60 pbw cobalt and 0.1-100 pbw of at least one other metal chosen from the group formed by zirconium, titanium, rhenium, ruthenium and chromium per 100 pbw silica, alumina, silica-alumina and/or titania.

4. A process as claimed in claim 2 or 3, in which the catalyst satisfies the relation:$\text{(3+4R) >} \frac{\text{L}}{\text{S}} \text{> (0.3+0.4R),}$ wherein
L is the total quantity of cobalt present on the catalyst, expressed as mg Co/ml catalyst,
S is the surface area of the catalyst, expressed as m²/ml catalyst, and
R is the weight ratio of the quantity of cobalt deposited on the catalyst by kneading to the total quantity of cobalt present on the catalyst.

5. A process as claimed in any one of claims 1 to 4, in which in step (a) the Fischer-Tropsch reaction is carried out at a temperature of 125-350 °C an a a pressure of 5-100 bar.

6. A process as claimed in any one of claims 1 to 5, in which in step (b) a catalyst is used comprising a catalytically active metal component and a zeolite.

7. A process as claimed in claim 6, wherein the catalytically active metal component comprises at least one metal of Groups VIB, VIIB and VIII of the Periodic System.

8. A process as claimed in claim 7, wherein the catalytically active metal component comprises at least one metal from the group consisting of Mo, W, Re, Fe, Ni, Co, Ru, Rh, Pd, Os, Ir and Pt.

9. A process as claimed in any one of claims 6-8, in which the zeolite has a pore diameter in the range of 0.5 to 1.0 nm.

10. A process as claimed in any one of claims 6-9, in which the zeolite has a silica:alumina ratio in the range of 5 to 200.

11. A process as claimed in any one of claims 6 to 10, in which the zeolite is zeolite Y, zeolite β, mordenite, or ZSM-20.

12. A process as claimed in any one of claims 1 to 11, in which step (b) is carried out at a temperature of 250-450 °C, a pressure of 5-50 bar, an hourly space velocity of 0.2-20 kg of hydrocarbon feed per kg of catalyst per hour and a hydrogen/hydrocarbon feed molar ratio of 1-50.

13. A process as claimed in any one of claims 1 to 12, in which step (c) is carried out by contacting at least part of the effluent of step (b) with a separatory molecular sieve capable of adsorbing normal hydrocarbons, whereby a product stream containing branched hydrocarbons is separated off.

14. A process as claimed in claim 10, in which the separatory molecular sieve has a pore diameter in the range from 0.3 to 0.8 nm.

15. A process as claimed in claim 1, in which the heavy paraffins-containing hydrocarbon mixture is separated into a heavy fraction boiling above 150 °C and a light fraction containing C₁₀-hydrocarbons, the heavy fraction is passed to the hydrocracking step and the light fraction is passed to the step (b).

16. A process for the production of isoparaffins having 4 to 7 carbon atoms per molecule from synthesis gas comprising the following steps:
a) Synthesis of a heavy paraffins-containing hydrocarbon mixture over a Fischer-Tropsch catalyst; separating the mixture into a heavy fraction boiling above 150 °C and a light fraction containing C10-hydrocarbons; passing the light fraction to step (b) and hydrocracking the heavy fraction in a hydrocracking step;
b) Conversion of the light fraction of the heavy paraffins-containing hydrocarbon mixture over a bi-functional isomerization/hydrocracking catalyst under isomerization/hydrocracking conditions;
Combining the effluents from the hydrocracking step and of the isomerization/hydrocracking step of step (b) and separating the combined effluents into a hydrogen-containing gas which is at least in part recycled to the hydrocracking step and/or the isomerization/hydrocracking step of step (b), a naphtha fraction which is at least in part passed to step (c), a kerosene fraction and a gas oil fraction which are withdrawn as products from the process, and a heavy fraction which is at least in part recycled to the hydrocracking step.
c) Separation of normal paraffins from at least part of the isoparaffins-containing naphtha fraction of step b) ;
d) Recycling at least part of the normal paraffins to the isomerization/hydrocracking step of step b) and withdrawing C₄-C₇ isoparaffins as a product from the process.

## Patentansprüche

1. Verfahren zur Herstellung von Isoparaffinen mit 4 bis 7 Kohlenstoffatomen pro Molekül aus Synthesegas, bei dem man:
a) an einem Fischer-Tropsch-Katalysator ein schwere Paraffine enthaltendes Kohlenwasserstoffgemisch synthetisiert; das schwere Paraffine enthaltende Kohlenwasserstoffgemisch in einem Hydrocrackreaktor mindestens teilweise hydrocrackt; den Austragsstrom des Hydrocrackreaktors in ein wasserstoffhaltiges Gas, das man mindestens teilweise in den Hydrocrackreaktor zurückführt, eine Naphthafraktion, die man mindestens teilweise dem Schritt (b) zuführt, eine Kerosin- und eine Gasölfraktion, die man als Verfahrensprodukte abzieht, und in eine schwere Fraktion, die man mindestens teilweise zum Hydrocrackschritt zurückführt, auf trennt;
b) die Naphthafraktion des Schritts (a) unter Isomerisierungs/Hydrocrack-Bedingungen mindestens teilweise an einem bifunktionellen Isomerisierungs-Hydrocrack-Katalysator umwandelt;
c) aus mindestens einem Teil des Isoparaffine enthaltenden Produkts aus Schritt (b) normal-Paraffine abtrennt;
d) die normal-Paraffine mindestens teilweise zum Schritt (b) zurückführt und C₄-C₇-Isoparaffine als Verfahrensprodukt abzieht.

2. Verfahren nach Anspruch 1, bei dem man in Schritt (a) einen Cobalt enthaltenden Fischer-Tropsch-Katalysator verwendet.

3. Verfahren nach Anspruch 2, wobei der Katalysator pro 100 Gew.-Teile Siliciumdioxid, Aluminiumoxid, Siliciumdioxid-Aluminiumoxid und/oder Titandioxid 3-60 Gew.-Teile Cobalt und 0,1-100 Gew.-Teile mindestens eines anderen Metalls, ausgewählt aus der Gruppe, gebildet aus Zirkonium, Titan, Rhenium, Ruthenium und Chrom, enthält.

4. Verfahren nach Anspruch 2 oder 3, wobei der Katalysator der Beziehung:$\text{(3+4R) >} \frac{\text{L}}{\text{S}} \text{> (0,3+0,4R)}$ genügt, worin
L die auf dem Katalysator vorliegende Gesamtmenge an Cobalt, ausgedrückt in mg Co/ml Katalysator,
S die Oberfläche des Katalysators, ausgedrückt als m²/ml Katalysator und
R das Gewichtsverhältnis zwischen der durch Verkneten auf dem Katalysator abgeschiedenen Menge an Cobalt und der auf dem Katalysator vorliegenden Gesamtmenge an Cobalt bedeutet.

5. Verfahren nach einem der Ansprüche 1 bis 4, bei dem man im Schritt (a) die Fischer-Tropsch-Reaktion bei einer Temperatur von 125-350°C und einem Druck von 5-100 bar durchführt.

6. Verfahren nach einem der Ansprüche 1 bis 5, bei dem man in Schritt (b) einen eine katalytisch aktive Metallkomponente und einen Zeolith enthaltenden Katalysator verwendet.

7. Verfahren nach Anspruch 6, wobei die katalytischh aktive Metallkomponente mindestens ein Metall aus den Gruppen VIB, VIIB und VIII des Periodensystems enthält.

8. Verfahren nach Anspruch 7, wobei die katalytisch aktive Metallkomponente mindestens ein Metall aus der Gruppe, bestehend aus Mo, W, Re, Fe, Ni, Co, Ru, Rh, Pd, Os, Ir und Pt, enthält.

9. Verfahren nach einem der Ansprüche 6-8, wobei der Zeolith einen Porendurchmesser im Bereich von 0,5 bis 1,0 nm aufweist.

10. Verfahren nach einem der Ansprüche 6-9, wobei der Zeolith ein Siliciumdioxid/Aluminiumoxid-Verhältnis im Bereich von 5 bis 200 aufweist.

11. Verfahren nach einem der Ansprüche 6 bis 10, bei dem man als Zeolith Y-Zeolith, β-Zeolith, Mordenit oder ZSM-20 einsetzt.

12. Verfahren nach einem der Ansprüche 1 bis 11, bei dem man den Schritt (b) bei einer Temperatur von 250-450°C, einem Druck von 5-50 bar, einer Katalysatorbelastung von 0,2-20 kg Kohlenwasserstoffeinsatz pro kg Katalysator pro Stunde und bei einem Wasserstoff/Kohlenwasserstoffeinsatz-Molverhältnis von 1-50 durchführt.

13. Verfahren nach einem der Ansprüche 1 bis 12, bei dem man Schritt (c) so durchführt, daß man den Austragsstrom des Schritts (b) wenigstens teilweise mit einem trennwirksamen Molekularsieb, das normal-Kohlenwasserstoffe adsorbieren kann, in Berührung bringt, wodurch ein verzweigte Kohlenwasserstoffe enthaltender Produktstrom abgetrennt wird.

14. Verfahren nach Anspruch 10, wobei das trennwirksame Molekularsieb einen Porendurchmesser im Bereich von 0,3 bis 0,8 nm aufweist.

15. Verfahren nach Anspruch 1, bei dem man das schwere Paraffine enthaltende Kohlenwasserstoffgemisch in eine über 150°C siedende schwere Fraktion und eine C₁₀-Kohlenwasserstoffe enthaltende leichte Fraktion auftrennt und die schwere Fraktion dem Hydrocrackschritt und die leichte Fraktion Schritt (b) zuführt.

16. Verfahren zur Herstellung von Isoparaffinen mit 4 bis 7 Kohlenstoffatomen pro Molekül aus Synthesegas, bei dem man:
a) an einem Fischer-Tropsch-Katalysator ein schwere Paraffine enthaltendes Kohlenwasserstoffgemisch synthetisiert, das Gemisch in eine schwere, über 150°C siedende Fraktion und eine leichte, C₁₀-Kohlenwasserstoffe enthaltende Fraktion auftrennt, die leichte Fraktion dem Schritt (b) zuführt und die schwere Fraktion in einem Hydrocrackschritt hydrocrackt;
b) die leichte Fraktion des schwere Paraffine enthaltenden Kohlenwasserstoffgemischs unter Isomerisierungs-Hydrocrack-Bedingungen an einem bifunktionellen Isomerisierungs-Hydrocrack-Katalysator umwandelt;
die Austragsströme des Hydrocrackschritts und des Isomerisierungs-Hydrocrack-Schritts aus Schritt (b) vereinigt und die vereinigten Austragsströme in ein wasserstoffhaltiges Gas, das man mindestens teilweise in den Hydrocrackschritt und/oder den Isomerisierungs-Hydrocrack-Schritt des Schritts (b) zurückführt, eine Gasölfraktion, die man als Verfahrensprodukte abzieht, und eine schwere Fraktion, die man mindestens teilweise zum Hydrocrackschritt zurückführt, auftrennt;
c) aus der Isoparaffine enthaltenden Naphthafraktion aus Schritt (b) mindestens teilweise normal-Paraffine abtrennt;
d) die normal-Paraffine mindestens teilweise zum Isomerisierungs-Hydrocrackschritt aus Schritt (b) zurückführt und C₄-C₇-Isoparaffine als Verfahrensprodukt abzieht.

## Revendications

1. Procédé de préparation d'isoparaffines possédant de 4 à 7 atomes de carbone par molécule à partir du gaz de synthèse, caractérisé en ce qu'il comprend les étapes suivantes:
a) Synthèse d'un mélange d'hydrocarbures contenant des paraffines lourds sur un catalyseur de Fischer-Tropsch; hydrocraquage d'au moins une partie du mélange d'hydrocarbures contenant des paraffines lourds dans un réacteur d'hydrocraquage; séparation de l'effluent du réacteur d'hydrocraquage en un gaz contenant de l'hydrogène qui est au moins en partie recyclé au réacteur d'hydrocraquage, une fraction de naphta qui est au moins en partie amenée à l'étape (b), une fraction de kérosène et une fraction de gazole qui sont prélevées sous forme de produits du procédé, et une fraction lourde qui est au moins en partie recyclée à l'étape d'hydrocraquage;
b) Conversion d'au moins une partie de la fraction de naphta de l'étape (a) sur un catalyseur d'isomérisation/hydrocraquage bifonctionnel dans des conditions d'isomérisation/hydrocraquage;
c) Séparation de paraffines normales d'au moins une partie du produit contenant des isoparaffines de l'étape (b);
d) Recyclage d'au moins une partie des paraffines normales à l'étape (b) et prélèvement des isoparaffines en C₄ à C₇ en tant que produit issu du procédé.

2. Procédé suivant la revendication 1, caractérisé en ce qu'au cours de l'étape (a), on utilise un catalyseur de Fisher-Tropsch, comprenant du cobalt.

3. Procédé suivant la revendication 2, caractérisé en ce que le catalyseur comprend 3 à 60 parties en poids de cobalt et 0,1 à 100 parties en poids d'au moins un métal choisi dans le groupe formé par le zirconium, le titane, le rhénium, le ruthénium et le chrome par 100 parties en poids de silice, alumine, silice-alumine et/ou oxyde de titane.

4. Procédé suivant la revendication 2 ou 3, caractérisé en ce que le catalyseur satisfait à la relation:$\text{(3+4R) >} \frac{\text{L}}{\text{S}} \text{> (0,3+0,4R),}$ dans laquelle
L représente la quantité totale de cobalt présent sur le catalyseur, exprimée sous forme de mg de Co/ml de catalyseur,
S représente la surface spécifique du catalyseur, exprimée en m²/ml de catalyseur et
R représente le rapport pondéral de la quantité de catalyseur déposée sur le catalyseur par malaxage à la quantité totale de cobalt présent sur le catalyseur.

5. Procédé suivant l'une quelconque des revendications 1 à 4, caractérisé en ce que dans l'étape (a), on réalise la réaction de Fischer-Tropsch à une température de 125 à 350°C et sous une pression de 5 à 100 bars.

6. Procédé suivant l'une quelconque des revendications 1 à 5, caractérisé en ce que, dans l'étape (b), on utilise un catalyseur qui comprend un composant de métal catalytiquement actif et une zéolite.

7. Procédé suivant la revendication 6, caractérisé en ce que le composant de métal catalytiquement actif comprend au moins un métal des groupes VIB, VIIB et VIII du système périodique.

8. Procédé suivant la revendication 7, caractérisé en ce que le composant de métal catalytiquement actif comprend au moins un métal choisi dans le groupe formé par les suivants : Mo, W, Re, Fe, Ni, Co, Ru, Rh, Pd, Os, Ir et Pt.

9. Procédé suivant l'une quelconque des revendications 6 à 8, caractérisé en ce que la zéolite possède un diamètre des pores qui varie de 0,5 à 1,0 nm.

10. Procédé suivant l'une quelconque des revendications 6 à 9, caractérisé en ce que la zéolite possède un rapport silice:alumine qui varie dans la plage de 5 à 200.

11. Procédé suivant l'une quelconque des revendications 6 à 10, caractérisé en ce que la zéolite est une zéolite Y, une zéolite β, une mordénite, ou une ZSM-20.

12. Procédé suivant l'une quelconque des revendications 1 à 11, caractérisé en ce que l'on entreprend l'étape (b) à une température de 250 à 450°C, une pression de 5 à 50 bars, une vitesse spatiale horaire de 0,2 à 20 kg de charge hydrocarbonée par kg de catalyseur et par heure et un rapport molaire hydrogène/charge hydrocarbonée de 1-50.

13. Procédé suivant l'une quelconque des revendications 1 à 12, caractérisé en ce que l'on entreprend l'étape (c) par la mise en contact d'au moins une partie de l'effluent de l'étape (b) avec un tamis moléculaire séparateur capable d'adsorber des hydrocarbures normaux, opération au cours de laquelle un courant de produit contenant des hydrocarbures ramifiés est séparé.

14. Procédé suivant la revendication 10, caractérisé en ce que le tamis moléculaire séparateur possède un diamètre des pores qui varie de 0,3 à 0,8 nm.

15. Procédé suivant la revendication 1, caractérisé en ce que le mélange d'hydrocarbures contenant des paraffines lourds est séparé en une fraction lourde bouillant au dessus de 150°C et une fraction légère contenant des hydrocarbures en C₁₀ et moins, on fait passer la fraction lourde dans l'étape d'hydrocraquage et on fait passer la fraction légère dans l'étape d).

16. Procédé de production d'isoparaffines comportant de 4 à 7 atomes de carbone par molécule à partir de gaz de synthèse, caractérisé en ce qu'il comprend les étapes suivantes:
a) Synthèse d'un mélange d'hydrocarbures contenant des paraffines lourds sur un catalyseur de Fischer-Tropsch, séparation du mélange en une fraction lourde bouillant au dessus de 150°C et une fraction légère contenant des hydrocarbures en C₁₀ et moins, le passage de la fraction légère à l'étape b) et l'hydrocraquage de la fraction lourde dans une étape d'hydrocraquage,
b) Conversion de la fraction légère du mélange d'hydrocarbures contenant des paraffines lourds sur un catalyseur d'isomérisation/hydrocraquage bifonctionnel dans des conditions d'isomérisation/hydrocraquage,
Combinaison des effluents de l'étape d'hydrocraquage et de l'étape d'isomérisation/hydrocraquage de l'étape (b) et séparation des effluents combinés en un gaz contenant de l'hydrogène qui est au moins en partie recyclé à l'étape d'hydrocraquage et/ou l'étape d'isomérisation/hydrocraquage de l'étape (b), une fraction de naphta que l'on fait, au moins en partie, passer à l'étape (c), une fraction de kérosène et une fraction de gazole qui sont soutirées comme produits issus du procédé et une fraction lourde qui est au moins en partie recyclée dans l'étape d'hydrocraquage,
c) Séparation des paraffines normales d'au moins une partie de la fraction de naphta contenant des isoparaffines de l'étape b),
d) Recyclage d'au moins une partie des paraffines normales à l'étape d'isomérisation/hydrocraquage de l'étape b) et prélèvement des isoparaffines en C₄ à C₇ à titre de produit issu du procédé.
